# EUROPEAN PATENT SPECIFICATION

(11) **EP 3 324 753 B1**
(45) Date of publication and mention of the grant of the patent: **06.12.2023**
(21) Application number: 16745370.3
(22) Date of filing: 22.07.2016
(51) Int. Cl.: A23K 50/10, A23K 50/30, A23K 50/75, A23K 10/37, A23K 20/158, A23K 20/105, A23K 20/163, A23K 20/24, A23K 20/20, A61K 31/19, A23K 20/10

(54) **ODOR FREE VOLATILE FATTY ACIDS AS AN ENERGY SOURCE FOR RUMINANTS, SWINE AND POULTRY**
GERUCHLOSE FLÜCHTIGEN FETTSÄUREN ALS ENERGIEQUELLE FÜR WIEDERKÄUER, SCHWEINE UND GEFLÜGEL
ACIDES GRAS VOLATILS INODORES COMME SOURCE D'ÉNERGIE POUR RUMINANTS, PORCS ET VOLAILLE

(30) Priority: 22.07.2015 US 201514805571; 02.09.2015 US 201514843130
(43) Date of publication of application: 30.05.2018
(73) Proprietor: Zinpro Corporation, Eden Prairie, MN 55344 (US)
(72) Inventor: STARK, Peter A., Eden Prairie, Minnesota 55344 (US); KENDING, Cory Shawn, Eden Prairie, Minnesota 55344 (US); SOCHA, Michael Thomas, Eden Prairie, Minnesota 55344 (US)
(74) Representative: Tomkinson, Alexandra
(86) International application number: PCT/US2016/043494
(87) International publication number: WO 2017/015537

(56) References cited:
- WO-A1-84/00668
- WO-A1-91/11915
- WO-A1-2014/047497
- US-A- 4 376 790

## Description

### CROSS-REFERENCE TO RELATED APPLICATIONS

This is a Continuation-in-part Application of U. S. Serial No. 14/805,571 filed July 22, 2015.

### FIELD OF THE INVENTION

Production and use of essentially odor free nutrients especially for ruminants as an energy source leading to increased milk production, but also as an odor free nutrient for swine and poultry.

### BACKGROUND OF THE INVENTION

It is well-known in the animal nutrition field that volatile fatty acids such as butyric acid, isobutyric acid, and valeric acid improve milk production in dairy cows. However one of the main drawbacks of using these volatile acids for this purpose is their strong odor. The odor has sometimes been described as smelling of extreme rancidity, vomit, and/or extreme body odor. Eastman Kodak originally produced these compounds for the animal industry, see U.S. Patent No. 4,804,547, which discloses making calcium salts of the isoacids, but they never saw widespread use, due to their odor. The odor was less a problem to the animals eating the fermentation enhancer than it was to the workers producing it. Oftentimes workers could not stand the smell, sickened and some even claimed adverse medical effects. There were some efforts to decrease odor, such as U.S. Patent No. 4,376,790, which relates to decreasing odor by making ammonium salts of the isoacids. Another attempt at improving this type of product was to make the imines from urea and corresponding acid aldehydes (see Publication No. WO 84/006769). However, the aldehydes are significantly more expensive than the acids and this therefore never became a viable product.

Isoacids is the collective term for the branched-chain fatty acids: isobutyric, 2-methylbutyric and isovaleric acid and the straight-chain valeric acid, which are naturally produced in ruminant's digestive tracts. They are mainly built up from the degradation products of the amino acids valine, isoleucine, leucine and proline and should in turn be used for the biosynthesis of those amino acids and higher branched chain volatile fatty acids. Besides their role as specific nutrients for the ruminal cellulolytic bacteria, isoacids seem to have a general positive influence on microbial fermentation. Only limited information is available on the influence of isoacids on the intermediary metabolism. Alteration of the growth hormone and indirect effects (via amino acids) on mammary gland and skeletal muscles are suggested. From a review of cattle experiments, a nutritional supplement of isoacids may also has a positive influence on milk production. For a scientific discussion of isoacides in the digestion and metabolism of the ruminant, see Animal Feed Science and Technology, 18 (1987) 169-180.

There is a continuing need for a convenient low-cost process to lower the odor so as to make volatile fatty acid derived fermentation enhancers a viable feed supplement product that can be used to increase milk production.

There are additional phenomena that are taken advantage in the present invention besides odor reduction. For example, sugars are known to have energy value in feeding ruminants. Typically, they are sticky and difficult to work with. Often they are delivered in liquid form. This invention can provide in some embodiments those as part of the fermentation enhancer composition in an easily processable, and dosable form.

In addition, macro minerals such as calcium and magnesium are important to both the microflora of the rumen, as well as the overall wellbeing of the animal.

There is therefore also a continuing need to develop an odor free fermentation enhancer to supplement ruminant feed that combines all three of these features to alleviate several problems associated with these feed ingredients in the past, all to make a viable product that can be used to increase milk production.

This invention has as its primary objective fulfillment of this continuing need.

WO2014/047497 discloses nutritional compositions including calcium beta-hydroxy-beta-methylbutyrate that has been treated by sequestration (chelation) and with an ion exchange resin/polymer so as to render the calcium ions less soluble in liquid compositions. The stability of such compositions is improved to allow them to be used to treat individuals with diabetes and chronic kidney dysfunction.

WO91/11915 discloses a supplemental feed pellet formed of a complex of polysaccharides of low ruminal solubility having suspended within the complex a chemical or combination of chemicals which will increase ruminal microbial activity over extended periods of time as the chemicals are slowly released into the ruminal fluids.

### SUMMARY OF THE INVENTION

According to one aspect of the present invention there is provided a feed supplement for ruminants, swine and poultry according to claim 1.

According to one aspect of the present invention there is provided a method of feeding ruminant animals, swine and poultry and energy source according to claim 6.

According to one aspect of the present invention there is provided a process of producing a reduced odor feed supplement containing isoacids according to claim 9.

This invention overcomes the odor problems of volatile fatty acid nutrients, and combines the advantages of processable sugars and macro minerals such as, calcium and magnesium, by preparing feed supplements for ruminants, swine and poultry from polycarboxylic acid metal salts and volatile fatty acids. Preferably, calcium and magnesium salts of pendant polycarboxylic acid groups derived from readily available materials, such as pectin are reacted with the volatile fatty acids to provide useful low odor nutrients.

### DETAILED DESCRIPTION OF THE PREFERRED EMBODIMENTS

The feed supplements that are essentially odor free and that enhance milk production are prepared by reacting a water soluble polycarboxylic acid, metal salt with pendant carboxylic acid groups and an isoacid selected from the group consisting of isobutyric, isovaleric, 2-methyl-butyric and valeric acids.

The water soluble polycarboxylic acid is selected from the group pectin, alginic acid and carboxymethyl cellulose.

The metal ions are selected from the group consisting of calcium, magnesium, zinc, manganese, copper and iron, but are most preferably calcium or magnesium.

The product may be dosed to the ruminant animals at from 10 grams per head to 100 grams per head, preferably from 20 grams per head to 80 grams per head per day. For swine and poultry it may be fed at 0.01% to 1% of feed ration, preferably 0.1% by weight of feed ration to 0.3% by weight of feed ration.

As illustrated in the examples below, the composition may be used on a dry as is basis (neat), or with the usual carriers or feed supplements such as corn cobs, whey, fermentation byproducts, soybean flour, soy meal, and barley, etc. It can also be used directly as a liquid.

While the primary emphasis of the description here has been for use with ruminants, the composition is also used as an energy source for swine and poultry industries as well, since butyric acid is sometimes used in these industries, and when so used has the same odor issue here described.

The following examples of preparation are shown to illustrate in an exemplary fashion various isoacids, various carriers, various metals, particularly calcium and magnesium, and examples of some product neat (without carrier and some products with carrier). In addition, the polymers used illustrate pectin, alginic acid, polyacrylic acid, and carboxymethylcellulose. The four volatile fatty acids used will be butyric, isobutyric, valeric and, 2-methyl-butyric acid. The structures illustrated are functionally shown below: 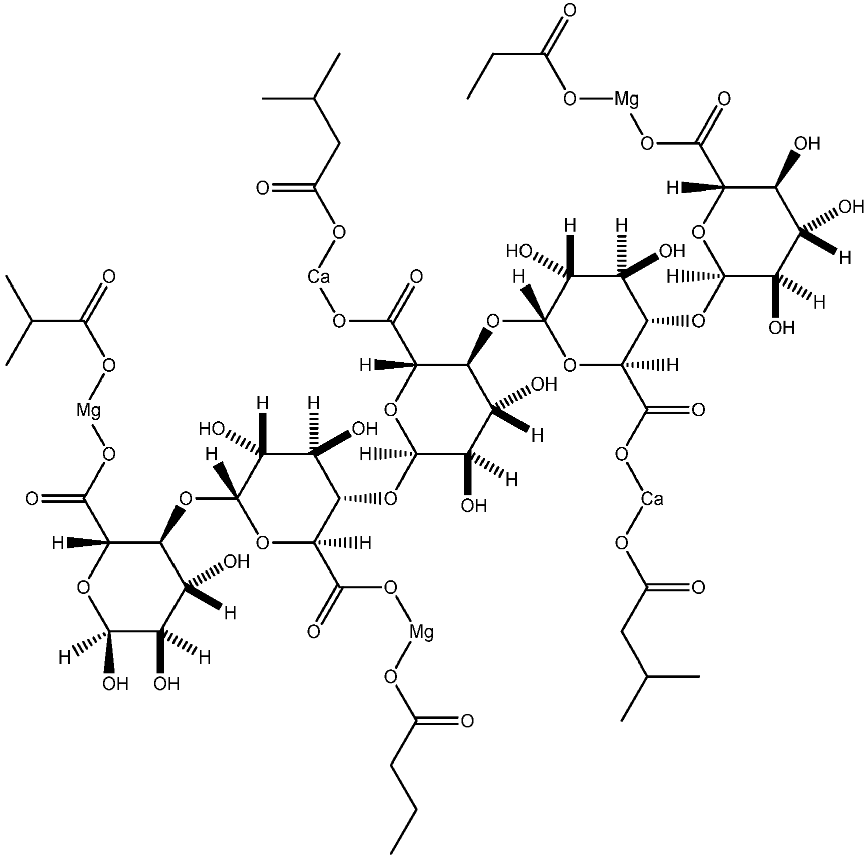 (pectin metal salt with volatile fatty acids (VFA))

For this first series of examples, i.e., 1 through 80, the only testing done on the samples was an odor test to show how this synthesis produces a reaction product of low odor. The odor testing was done blind, according to the following methodology.

### Odor Testing Protocol:

### The Odor Assessor:

1. Must be free of colds or physical conditions that may affect the sense of smell;
2. Must not chew gum or eat at least 30 minutes prior to the test;
3. Must refrain from eating spicy foods prior to the test; and
4. Must not wear perfume cologne or after shave the day of the test.

### The Odor Intensity Test:

During an odor test, the odor assessor, sniffs a sample of the product as the container is opened approximately six inches directly below the assessor's nostrils. The odor intensity is then qualitatively compared to control samples; isobutyric acid (IBA), 1:10 IBA/water, 1:100 IBA/water and pure water. A score of zero odor units indicates no smell. A score of three odor units indicates an odor intensity equivalent to 1:100 IBA/water. A score of six odor units indicates an odor intensity equivalent to 1: 10 IBA/water. A score often odor units indicates an odor as intense as undiluted isobutyric acid. The test is repeated as necessary with the assessor revisiting the controls and test product as often as necessary prior to a qualitative confidence being reached. The assessor then repeats this test on a series of no greater than ten individual test products in one 24 hour period. The individual estimated intensities for three to five assessments and are averaged to the nearest whole number to determine the reportable odor intensity. Some of the Examples are of ingredients other than the invention to provide very smelly comparisons or controls. These are indicated as (comparative).

### Example 1

### MgCl₂ - mixed VFA - Pectin - NaOH

Pectin (10.02g, 51 mmols), was suspended in 100mL of 2M NaOH (8.01g, 0.2mols) and heated to 70°C for 4 hours. Isobutyric Acid (1.39g, 15.8mmols), Isovaleric Acid (1.01g, 9.89mmols), 2-MethylButyric Acid (1.41g, 12.9mmols) and Valeric Acid (1.27g, 12.4mmols) were added in one portion and after 10 minutes of stirring Magnesium Chloride Hexahydrate (10.38g, 51mmols) in 5mL of water was slowly added to the resulting reddish orange suspension. After stirring for an additional hour to ensure homogeneity the burnt red suspension was dried in a vacuum oven and analyzed for smell.

### Smell Factor: 2

### Example 2

### MgCl₂ - mixed VFA - Pectin - NaOH - Corn Cob

Pectin (10.00g, 51 mmols), was suspended in 100mL of 2M NaOH (7.98g, 0.2mols) and heated to 70°C for 4 hours. Isobutyric Acid (1.41g, 15.8mmols), Isovaleric Acid (1.00g, 9.89mmols), 2-MethylButyric Acid (1.31g, 12.9mmols) and Valeric Acid (1.27g, 12.4mmols) were added in one portion and after 10 minutes of stirring Magnesium Chloride Hexahydrate (10.35g, 51mmols) in 5mL of water was slowly added to the resulting reddish orange suspension. After stirring for an additional hour to ensure homogeneity the burnt red suspension was added to 33g of Ground Corn Cob and dried in a vacuum oven prior to smell analysis.

### Smell Factor: 1

### Example 3

### MgCl₂ - Isobutyric Acid -CMC High Viscosity -KOH- Corn Cob

Sodium Carboxymethyl Cellulose - High Viscosity (2.00g, ~ 7.27mmols of COOH) was dissolved in 100mL of 2M KOH (11.21g, 0.2mols) over 4 hours. To this was added Isobutyric Acid (0.67mL, 7.27mmols) and the resulting suspension was stirred for 20 minutes and to this was added Magnesium Chloride Hexahydrate (1.48g, 7.27mmols). The homogenous suspension was then stirred for an additional hour, added to 33g of Ground Corn Cob and dried in a vacuum oven prior to analyzing for smell.

### Smell Factor: 1

### Example 4

### MgCl₂ - Isobutyric Acid -CMC - High Viscosity -KOH

Sodium Carboxymethyl Cellulose - High Viscosity (2.03g, ~ 7.27mmols of COOH) was dissolved in 100mL of 2M KOH (11.20g, 0.2mols) over 4 hours. To this was added Isobutyric Acid (0.67mL, 7.27mmols) and the resulting suspension was stirred for 20 minutes and to this was added Magnesium Chloride Hexahydrate (1.50g, 7.27mmols). The homogenous suspension was then stirred for an additional hour, evaporated and dried completely in a vacuum oven.

### Smell Factor: 1

### Example 5

### MgCl₂ - Valeric/IsoValeric -CMC High Viscosity -KOH- Corn Cob

Sodium Carboxymethyl Cellulose - High Viscosity (1.96g, ~ 7.27mmols of COOH) was dissolved in 100mL of 2M KOH (11.18g, 0.2mols) over 4 hours. To this was added both Valeric Acid (0.39mL, 3.64 mmols) and Isovaleric Acid (0.39mL, 3.64mmols) the resulting suspension was stirred for 20 minutes and to this was added Magnesium Chloride Hexahydrate (1.49g, 7.27mmols). The homogenous suspension was then stirred for an additional hour, added to 33g of Ground Corn Cob and dried in a vacuum oven prior to analyzing for smell.

### Smell Factor: 1

### Example 6

### MgCl₂ - Valeric/IsoValeric -CMC High Viscosity -KOH

Sodium Carboxymethyl Cellulose - High Viscosity (1.99g, ~ 7.27mmols of COOH) was dissolved in 100mL of 2M KOH (11.20g, 0.2mols) over 4 hours. To this was added both Valeric Acid (0.39mL, 3.64 mmols) and Isovaleric Acid (0.39mL, 3.64mmols) the resulting suspension was stirred for 20 minutes and to this was added Magnesium Chloride Hexahydrate (1.45g, 7.27mmols). The homogenous suspension was then stirred for an additional hour, evaporated and dried completely in a vacuum oven.

### Smell Factor: 1

### Example 7

### MgCl₂ - Valeric/IsoValeric/2-Methyl Butyric -CMC High Viscosity -KOH- Corn Cob

Sodium Carboxymethyl Cellulose - High Viscosity (1.97g, ~ 7.27mmols of COOH) was dissolved in 100mL of 2M KOH (11.21g, 0.2mols) over 4 hours. To this was added Valeric Acid (0.27mL, 2.42 mmols), Isovaleric Acid (0.27mL, 2.42mmols) and 2-methyl butryric acid (0.27mL, 2.42mmols) the resulting suspension was stirred for 20 minutes and to this was added Magnesium Chloride Hexahydrate (1.50g, 7.27mmols). The homogenous suspension was then stirred for an additional hour, added to 33g of Ground Corn Cob and dried in a vacuum oven prior to analyzing for smell.

### Smell Factor: 1

### Example 8

### MgCl₂ - Valeric/IsoValeric/2-Methyl Butyric -CMC High Viscosity -KOH

Sodium Carboxymethyl Cellulose - High Viscosity (1.96g, ~ 7.27mmols of COOH) was dissolved in 100mL of 2M KOH (11.18g, 0.2mols) over 4 hours. To this was added Valeric Acid (0.27mL, 2.42 mmols), Isovaleric Acid (0.27mL, 2.42mmols) and 2-methyl butryric acid (0.27mL, 2.42mmols) the resulting suspension was stirred for 20 minutes and to this was added Magnesium Chloride Hexahydrate (1.49g, 7.27mmols). The homogenous suspension was then stirred for an additional hour, evaporated and dried completely in a vacuum oven.

### Smell Factor: 1

### Example 9 (not according to the invention)

### MgCl₂ - 2-Methyl Butyric acid - Polyacrylic Acid - KOH

Polyacrylic Acid (50% solution, 7.61g, 51mmols), was dissolved in 50mL of 2M KOH (5.56g, 0.1mols). 2-methyl butyric acid (5.63mL, 51mmols) was added which was followed by the dropwise addition of Magnesium Chloride Hexahydrate (10.31g, 51mmol) dissolved in 5mL of water. The resulting white suspension was stirred for an additional hour until homogenous, dried in a vacuum oven and analyzed for smell.

### Smell Factor: 1

### Example 10 (not according to the invention)

### MgCl₂ - 2-Methyl Butyric acid - Polyacrylic Acid - KOH - Corn Cob

Polyacrylic Acid (50% solution, 7.55g, 51mmols), was dissolved in 50mL of 2M KOH (5.57g, 0.1mols). 2-methyl butyric acid (5.63mL, 51mmols) was added which was followed by the dropwise addition of Magnesium Chloride Hexahydrate (10.38g, 51mmol) dissolved in 5mL of water. The resulting white suspension was stirred for an additional hour until homogenous, added to 17g of Ground Corn Cob and dried in the vacuum oven prior to smell analysis.

### Smell Factor: 2

### Example 11 (not according to the invention)

### MgCl₂ - Isobutyric/2-methyl butyric acid - Polyacrylic Acid - KOH

Polyacrylic Acid (50% solution, 7.56g, 51mmols), was dissolved in 50mL of 2M KOH (5.57g, 0.1mols). Isobutyric (2.37mL, 25.5mmols) and 2-methyl butyric acid (2.82g, 25.5mmols) were added which was followed by the dropwise addition of Magnesium Chloride Hexahydrate (10.30g, 51mmol) dissolved in 5mL of water. The resulting white suspension was stirred for an additional hour until homogenous, dried in a vacuum oven and analyzed for smell.

### Smell Factor: 1

### Example 12 (not according to the invention)

### MgCl₂ - Isobutyric/2-methyl butyric acid - Polyacrylic Acid - KOH - Corn Cob

Polyacrylic Acid (50% solution, 7.53g, 51mmols), was dissolved in 50mL of 2M KOH (5.55g, 0.1mols). Isobutyric (2.37mL, 25.5mmols) and 2-methyl butyric acid (2.82g, 25.5mmols) were added which was followed by the dropwise addition of Magnesium Chloride Hexahydrate (10.36g, 51mmol) dissolved in 5mL of water. The resulting white suspension was stirred for an additional hour until homogenous, added to 17g of Ground Corn Cob and dried in the vacuum oven prior to smell analysis.

### Smell Factor: 2

### Example 13 (not according to the invention)

### MgCl₂ - Isobutyric/Valeric/IsoValeric Acid - Polyacrylic Acid - KOH

Polyacrylic Acid (50% solution, 7.50g, 51mmols), was dissolved in 50mL of 2M KOH (5.60g, 0.1mols). Isobutyric (1.58mL, 17mmols), Valeric (1.86mL, 17mmols) and Isovaleric (1.87mL, 17mmols) were added which was followed by the dropwise addition of Magnesium Chloride Hexahydrate (10.37g, 51mmol) dissolved in 5mL of water. The resulting white suspension was stirred for an additional hour until homogenous, dried in a vacuum oven and analyzed for smell.

### Smell Factor: 1

### Example 14 (not according to the invention) MgCl₂ - Isobutyric/Valeric/IsoValeric Acid - Polyacrylic Acid - KOH - Corn Cob

Polyacrylic Acid (50% solution, 7.55g, 51mmols), was dissolved in 50mL of 2M KOH (5.59g, 0.1mols). Isobutyric (1.58mL, 17mmols), Valeric (1.86mL, 17mmols) and Isovaleric (1.87mL, 17mmols) were added which was followed by the dropwise addition of Magnesium Chloride Hexahydrate (10.33g, 51mmol) dissolved in 5mL of water. The resulting white suspension was stirred for an additional hour until homogenous, added to 17g of Ground Corn Cob and dried in the vacuum oven prior to smell analysis.

### Smell Factor: 2

### Example 15

### MgCl₂ - Isobutyric Acid - Pectin - KOH

Pectin (10.01g, 51 mmols), was suspended in 100mL of 2M KOH (11.17g, 0.2mol) and heated to 70°C for 4 hours. Isobutyric Acid (4.73mL, 51mmols) was added in one portion and after 10 minutes of stirring Magnesium Chloride Hexahydrate (10.35g, 51mmols) in 5mL of water was slowly added to the resulting reddish orange suspension. After stirring for an additional hour to ensure homogeneity the burnt red suspension was dried in a vacuum oven and analyzed for smell.

### Smell Factor: 1

### Example 16

### MgCl₂ - Isobutyric Acid - Pectin - KOH- Corn Cob

Pectin (9.97g, 51 mmols), was suspended in 100mL of 2M KOH (11.19g, 0.2mol) and heated to 70°C for 4 hours. Isobutyric Acid (4.73mL, 51mmols) was added in one portion and after 10 minutes of stirring Magnesium Chloride Hexahydrate (10.40g, 51mmols) in 5mL of water was slowly added to the resulting reddish orange suspension. After stirring for an additional hour to ensure homogeneity the burnt red suspension was added to 33g of Ground Corn Cob and dried in a vacuum oven prior to smell analysis.

### Smell Factor: 1

### Example 17

### MgCl₂ - Valeric / Isovaleric Acid - Pectin - KOH

Pectin (10.00g, 51 mmols), was suspended in 100mL of 2M KOH (11.15g, 0.2mol) and heated to 70°C for 4 hours. Valeric (2.78mL, 25.5mmols) and Isovaleric (2.81mL, 25.5mmols) were both added in one portion and after 10 minutes of stirring Magnesium Chloride Hexahydrate (10.39g, 51mmols) in 5mL of water was slowly added to the resulting reddish orange suspension. After stirring for an additional hour to ensure homogeneity the burnt red suspension was dried in a vacuum oven and analyzed for smell.

### Smell Factor: 1

### Example 18

### MgCl₂ - Valeric / Isovaleric Acid - Pectin - KOH - Corn Cob

Pectin (10.03g, 51 mmols), was suspended in 100mL of 2M KOH (11.21g, 0.2mol) and heated to 70°C for 4 hours. Valeric (2.78mL, 25.5mmols) and Isovaleric (2.81mL, 25.5mmols) were both added in one portion and after 10 minutes of stirring Magnesium Chloride Hexahydrate (10.37g, 51mmols) in 5mL of water was slowly added to the resulting reddish orange suspension. After stirring for an additional hour to ensure homogeneity the burnt red suspension was added to 33g of Ground Corn Cob and dried in a vacuum oven prior to smell analysis.

### Smell Factor: 1

### Example 19

### MgCl₂ - Isobutyric/Isovaleric/2-Methyl-Butyric Acid - Pectin - KOH

Pectin (9.95g, 51 mmols), was suspended in 100mL of 2M KOH (11.15g, 0.2mol) and heated to 70°C for 4 hours. Isobutyric (1.58mL, 17mmols), Isovaleric (1.87mL, 17mmols) and 2-methyl butyric (1.88mL) were added in one portion and after 10 minutes of stirring Magnesium Chloride Hexahydrate (10.34g, 51mmols) in 5mL of water was slowly added to the resulting reddish orange suspension. After stirring for an additional hour to ensure homogeneity the burnt red suspension was dried in a vacuum oven and analyzed for smell.

### Smell Factor: 1

### Example 20

### MgCl₂ - Isobutyric/Isovaleric/2-Methyl-Butyric Acid - Pectin - KOH - Corn Cob

Pectin (10.02g, 51 mmols), was suspended in 100mL of 2M KOH (11.25g, 0.2mol) and heated to 70°C for 4 hours. Isobutyric (1.58mL, 17mmols), Isovaleric (1.87mL, 17mmols) and 2-methyl butyric (1.88mL, 17mmols) were added in one portion and after 10 minutes of stirring Magnesium Chloride Hexahydrate (10.33g, 51mmols) in 5mL of water was slowly added to the resulting reddish orange suspension. After stirring for an additional hour to ensure homogeneity the burnt red suspension was added to 33g of Ground Corn Cob and dried in a vacuum oven prior to smell analysis.

### Smell Factor: 1

### Example 21

### MgCl₂ - mixed VFA - CMC (Sodium Salt) - High Viscosity

Sodium Carboxymethyl Cellulose - High Viscosity (2.01g, ~ 7.27mmols of COOH) was dissolved in 100mL of H₂O over 24 hours. To this was added Isobutyric Acid (198.5mg, 2.25mmols), Isovaleric Acid (144.2mg, 1.41mmols), 2-MethylButyric Acid (187.4mg, 1.83mmols) and Valeric Acid (181.4mg, 1.77mmols) the resulting suspension was stirred for 20 minutes and to this was added Magnesium Chloride Hexahydrate (1.45g, 7.27mmols). The homogenous suspension was then stirred for an additional hour, dried in the vacuum oven and analyzed for smell.

### Smell Factor: 1

### Example 22

### MgCl₂ - mixed VFA - CMC (Sodium Salt) - High Viscosity - Corn Cob

Sodium Carboxymethyl Cellulose - High Viscosity (1.97g, ~ 7.27mmols of COOH) was dissolved in 100mL of H₂O over 24 hours. To this was added Isobutyric Acid (198.5mg, 2.25mmols), Isovaleric Acid (144.2mg, 1.41mmols), 2-MethylButyric Acid (187.4mg, 1.83mmols) and Valeric Acid (181.4mg, 1.77mmols) the resulting suspension was stirred for 20 minutes and to this was added Magnesium Chloride Hexahydrate (1.47g, 7.27mmols). The homogenous suspension was then stirred for an additional hour, dried in the vacuum oven and analyzed for smell. The homogenous suspension was then stirred for an additional hour, added to 33g of Ground Corn Cob and dried in a vacuum oven prior to analyzing for smell.

### Smell Factor: 1

### Example 23

### MgCl₂ - mixed VFA - CMC - High Viscosity - KOH

Sodium Carboxymethyl Cellulose - High Viscosity (1.99g, ~ 7.27mmols of COOH) was dissolved in 100mL of 2M KOH (11.11g, 0.2mols) over 4 hours. To this was added Isobutyric Acid (198.5mg, 2.25mmols), Isovaleric Acid (144.2mg, 1.41mmols), 2-MethylButyric Acid (187.4mg, 1.83mmols) and Valeric Acid (181.4mg, 1.77mmols) the resulting suspension was stirred for 20 minutes and to this was added Magnesium Chloride Hexahydrate (1.51g, 7.27mmols). The homogenous suspension was then stirred for an additional hour, added to 33g of Ground Corn Cob and dried in a vacuum oven prior to analyzing for smell.

### Smell Factor: 2

### Example 24

### MgCl₂ - mixed VFA - CMC - High Viscosity - KOH - Corn Cob

Sodium Carboxymethyl Cellulose - High Viscosity (2.05g, ~ 7.27mmols of COOH) was dissolved in 100mL of 2M KOH (11.15g, 0.2mols) over 4 hours. To this was added Isobutyric Acid (198.5mg, 2.25mmols), Isovaleric Acid (144.2mg, 1.41mmols), 2-MethylButyric Acid (187.4mg, 1.83mmols) and Valeric Acid (181.4mg, 1.77mmols) the resulting suspension was stirred for 20 minutes and to this was added Magnesium Chloride Hexahydrate (1.43g, 7.27mmols). The homogenous suspension was then stirred for an additional hour, added to 33g of Ground Corn Cob and dried in a vacuum oven prior to analyzing for smell.

### Smell Factor: 1

### Example 25

### MgCl₂ - mixed VFA - CMC - Medium Viscosity

Sodium Carboxymethyl Cellulose - Medium Viscosity (2.03g, ~ 6.36mmols of COOH) was dissolved in 100mL of H₂O over 24 hours. To this was added Isobutyric Acid (173.7mg, 1.97mmols), Isovaleric Acid (126.2mg, 1.23mmols), 2-MethylButyric Acid (163.9mg, 1.60mmols) and Valeric Acid (158.7mg, 1.55mmols) the resulting suspension was stirred for 20 minutes and to this was added Magnesium Chloride Hexahydrate (1.29g, 6.4mmols). The homogenous suspension was then stirred for an additional hour, dried in the vacuum oven and analyzed for smell.

### Smell Factor: 1

### Example 26

### MgCl₂ - mixed VFA - CMC - Medium Viscosity - Corn Cob

Sodium Carboxymethyl Cellulose - Medium Viscosity (2.02g, ~ 6.36mmols of COOH) was dissolved in 100mL of H₂O over 24 hours. To this was added Isobutyric Acid (173.7mg, 1.97mmols), Isovaleric Acid (126.2mg, 1.23mmols), 2-MethylButyric Acid (163.9mg, 1.60mmols) and Valeric Acid (158.7mg, 1.55mmols) the resulting suspension was stirred for 20 minutes and to this was added Magnesium Chloride Hexahydrate (1.27g, 6.4mmols). The homogenous suspension was then stirred for an additional hour, added to 33g of Ground Corn Cob and dried in the vacuum oven prior to analyzing for smell.

### Smell Factor: 1

### Example 27

### MgCl₂ - mixed VFA - CMC - Medium Viscosity - KOH

Sodium Carboxymethyl Cellulose - Medium Viscosity (2.00g, ~ 6.36mmols of COOH) was dissolved in 100mL of 2M KOH (11.14g, 0.2mols) over 4 hours. To this was added Isobutyric Acid (173.7mg, 1.97mmols), Isovaleric Acid (126.2mg, 1.23mmols), 2-MethylButyric Acid (163.9mg, 1.60mmols) and Valeric Acid (158.7mg, 1.55mmols) the resulting suspension was stirred for 20 minutes and to this was added Magnesium Chloride Hexahydrate (1.25g, 6.4mmols). The homogenous suspension was then stirred for an additional hour, dried in the vacuum oven and analyzed for smell.

### Smell Factor: 1

### Example 28

### MgCl₂ - mixed VFA - CMC - Medium Viscosity - KOH - Corn Cob

Sodium Carboxymethyl Cellulose - Medium Viscosity (1.98g, ~ 6.36mmols of COOH) was dissolved in 100mL of 2M KOH (11.18g, 0.2mols) over 4 hours. To this was added Isobutyric Acid (173.7mg, 1.97mmols), Isovaleric Acid (126.2mg, 1.23mmols), 2-MethylButyric Acid (163.9mg, 1.60mmols) and Valeric Acid (158.7mg, 1.55mmols) the resulting suspension was stirred for 20 minutes and to this was added Magnesium Chloride Hexahydrate (1.30g, 6.4mmols). The homogenous suspension was then stirred for an additional hour, added to 33g of Ground Corn Cob and dried in the vacuum oven prior to analyzing for smell.

### Smell Factor: 1

### Example 29

### MgCl₂ - mixed VFA - CMC - Low Viscosity

Sodium Carboxymethyl Cellulose - Low Viscosity (4.03g, ~ 10.8mmols of COOH) was dissolved in 100mL of H₂O over 24 hours. To this was added Isobutyric Acid (295mg, 3.35mmols), Isovaleric Acid (214.3mg, 2.10mmols), 2-MethylButyric Acid (278.4mg, 2.72mmols) and Valeric Acid (269.6mg, 2.63mmols) the resulting suspension was stirred for 20 minutes and to this was added Magnesium Chloride Hexahydrate (2.20g, 11mmols). The homogenous suspension was then stirred for an additional hour, dried in the vacuum oven and analyzed for smell.

### Smell Factor: 1

### Example 30

### MgCl₂ - mixed VFA - CMC - Low Viscosity - Corn Cob

Sodium Carboxymethyl Cellulose - Low Viscosity (3.98g, ~ 10.8mmols of COOH) was dissolved in 100mL of H₂O over 24 hours. To this was added Isobutyric Acid (295mg, 3.35mmols), Isovaleric Acid (214.3mg, 2.10mmols), 2-MethylButyric Acid (278.4mg, 2.72mmols) and Valeric Acid (269.6mg, 2.63mmols) the resulting suspension was stirred for 20 minutes and to this was added Magnesium Chloride Hexahydrate (2.15g, 11mmols). The homogenous suspension was then stirred for an additional hour, added to 33g of Ground Corn Cob and dried in the vacuum oven prior to analyzing for smell.

### Smell Factor: 1

### Example 31

### MgCl₂ - mixed VFA - CMC - Low Viscosity - KOH

Sodium Carboxymethyl Cellulose - Low Viscosity (4.05g, ~ 10.8mmols of COOH) was dissolved in 100mL 2M KOH (11.19g, 0.2mols) over 2 hours. To this was added Isobutyric Acid (295mg, 3.35mmols), Isovaleric Acid (214.3mg, 2.10mmols), 2-MethylButyric Acid (278.4mg, 2.72mmols) and Valeric Acid (269.6mg, 2.63mmols) the resulting suspension was stirred for 20 minutes and to this was added Magnesium Chloride Hexahydrate (2.22g, 11mmols). The homogenous suspension was then stirred for an additional hour, dried in the vacuum oven and analyzed for smell.

### Smell Factor: 1

### Example 32

### MgCl₂ - mixed VFA - CMC - low Viscosity - KOH - Corn Cob

Sodium Carboxymethyl Cellulose - Low Viscosity (3.95g, ~ 10.8mmols of COOH) was dissolved in 100mL 2M KOH (11.14g, 0.2mols) over 2 hours. To this was added Isobutyric Acid (295mg, 3.35mmols), Isovaleric Acid (214.3mg, 2.10mmols), 2-MethylButyric Acid (278.4mg, 2.72mmols) and Valeric Acid (269.6mg, 2.63mmols) the resulting suspension was stirred for 20 minutes and to this was added Magnesium Chloride Hexahydrate (2.19g, 11mmols). The homogenous suspension was then stirred for an additional hour, added to 33g of Ground Corn Cob and dried in the vacuum oven prior to analyzing for smell.

### Smell Factor: 1

### Example 33 (Comparative)

### MgCl₂ - mixed VFA - Alginic Acid - Corn Cob

Alginic Acid (10.01g, 51mmols) was dissolved in 50mL of water and heated to 70°C for 4 hours. To this was added Isobutyric Acid (1.39g, 15.8mmols), Isovaleric Acid (1.01g, 9.89mmols), 2-MethylButyric Acid (1.31g, 12.9mmols) and Valeric Acid (1.27g, 12.4mmols) the resulting orange/yellow suspension was stirred for 30 minutes and to this the Magnesium Chloride Hexahydrate (10.33g, 51mmols) was added. The homogenous suspension was stirred for an additional hour, added to 17g of Ground Corn Cob and dried in the vacuum oven prior to analyzing for smell.

### Smell Factor: 4

### Example 34 (Comparative)

### MgCl₂ - mixed VFA - Alginic Acid (Neat)

Alginic Acid (9.97g, 51mmols) was dissolved in 50mL of water and heated to 70°C for 4 hours. To this was added Isobutyric Acid (1.39g, 15.8mmols), Isovaleric Acid (1.01g, 9.89mmols), 2-MethylButyric Acid (1.31g, 12.9mmols) and Valeric Acid (1.27g, 12.4mmols) the resulting orange/yellow suspension was stirred for 30 minutes and to this the Magnesium Chloride Hexahydrate (10.38g, 51mmols) was added. The homogenous suspension was stirred for an additional hour dried in the vacuum oven and analyzed for smell.

### Smell Factor: 5

### Example 35

### MgCl₂ - mixed VFA - Alginic Acid - KOH

Alginic Acid (9.98g, 51mmols) was dissolved in 50mL of 2M KOH (5.56g, 0.1mols) and heated to 70°C for 4 hours. To this was added Isobutyric Acid (1.39g, 15.8mmols), Isovaleric Acid (1.01g, 9.89mmols), 2-MethylButyric Acid (1.31g, 12.9mmols) and Valeric Acid (1.27g, 12.4mmols) the resulting orange/yellow suspension was stirred for 30 minutes and to this the Magnesium Chloride Hexahydrate (10.37g, 51mmols) was added. The homogenous suspension was stirred for an additional hour dried in the vacuum oven and analyzed for smell.

### Smell Factor: 1

### Example 36

### MgCl₂ - mixed VFA - Alginic Acid - KOH - Corn Cob

Alginic Acid (9.95g, 51mmols) was dissolved in 50mL 2M KOH (5.56g, 0.1mols) and heated to 70°C for 4 hours. To this was added Isobutyric Acid (1.42g, 15.8mmols), Isovaleric Acid (1.01g, 9.89mmols), 2-MethylButyric Acid (1.31g, 12.9mmols) and Valeric Acid (1.26g, 12.4mmols) the resulting orange/yellow suspension was stirred for 30 minutes and to this the Magnesium Chloride Hexahydrate (10.33g, 51mmols) was added. The homogenous suspension was stirred for an additional hour, added to 17g of Ground Corn Cob and dried in the vacuum oven prior to analyzing for smell.

### Smell Factor: 2

### Example 37 (Comparative)

### MgCl₂ - mixed VFA - Polyacrylic Acid

Polyacrylic Acid (50% solution, 7.60g, 51mmols), was dissolved in 50mL of water. Isobutyric Acid (1.39g, 15.8mmols), Isovaleric Acid (1.01g, 9.89mmols), 2-MethylButyric Acid (1.32g, 12.9mmols) and Valeric Acid (1.25g, 12.4mmols) were added which was followed by the dropwise addition of Magnesium Chloride Hexahydrate (10.40g, 51mmol) dissolved in 5mL of water. The resulting white suspension was stirred for an additional hour until homogenous, dried in a vacuum oven and analyzed for smell.

### Smell Factor: 7

### Example 38 (Comparative)

### MgCl₂ - mixed VFA - Polyacrylic Acid - Corn Cob

Polyacrylic Acid (50% solution, 7.57g, 51mmols), was dissolved in 50mL of water. Isobutyric Acid (1.40g, 15.8mmols), Isovaleric Acid (1.01g, 9.89mmols), 2-MethylButyric Acid (1.31g, 12.9mmols) and Valeric Acid (1.22g, 12.4mmols) were added which was followed by the dropwise addition of Magnesium Chloride Hexahydrate (10.36g, 51mmol) dissolved in 5mL of water. The resulting white suspension was stirred for an additional hour until homogenous, added to 17g of Ground Corn Cob and dried in the vacuum oven prior to smell analysis.

### Smell Factor: 6

### Example 39 (not according to the invention)

### MgCl₂ - mixed VFA - Polyacrylic Acid - KOH

Polyacrylic Acid (50% solution, 7.50g, 51mmols), was dissolved in 50mL of 2M KOH (5.54g, 0.1mols). Isobutyric Acid (1.39g, 15.8mmols), Isovaleric Acid (0.99g, 9.89mmols), 2-MethylButyric Acid (1.31g, 12.9mmols) and Valeric Acid (1.21g, 12.4mmols) were added which was followed by the dropwise addition of Magnesium Chloride Hexahydrate (10.34g, 51mmol) dissolved in 5mL of water. The resulting white suspension was stirred for an additional hour until homogenous, dried in a vacuum oven and analyzed for smell.

### Smell Factor: 3

### Example 40 (not according to the invention)

### MgCl₂ - mixed VFA - Polyacrylic Acid - KOH - Corn Cob

Polyacrylic Acid (50% solution, 7.55g, 51mmols), was dissolved in 50mL of 2M KOH (5.59g, 0.1mols). Isobutyric Acid (1.33g, 15.8mmols), Isovaleric Acid (1.01g, 9.89mmols), 2-MethylButyric Acid (1.31g, 12.9mmols) and Valeric Acid (1.27g, 12.4mmols) were added which was followed by the dropwise addition of Magnesium Chloride Hexahydrate (10.31g, 51mmol) dissolved in 5mL of water. The resulting white suspension was stirred for an additional hour until homogenous, added to 17g of Ground Corn Cob and dried in the vacuum oven prior to smell analysis.

### Smell Factor: 2

### Example 41

### MgCl₂ - mixed VFA - Pectin - KOH

Pectin (10.04g, 51 mmols), was suspended in 50mL of 4M KOH (11.12g, 0.2mols) and heated to 70°C for 4 hours. Isobutyric Acid (1.39g, 15.8mmols), Isovaleric Acid (1.00g, 9.89mmols), 2-MethylButyric Acid (1.30g, 12.9mmols) and Valeric Acid (1.30g, 12.4mmols) were added in one portion and after 10 minutes of stirring Magnesium Chloride Hexahydrate (10.36g, 51mmols) in 5mL of water was slowly added to the resulting reddish orange suspension. After stirring for an additional hour to ensure homogeneity the burnt red suspension was dried in a vacuum oven and analyzed for smell.

### Smell Factor: 2

### Example 42

### MgCl₂ - mixed VFA - Pectin - KOH - Ground Corn Cob

Pectin (9.94g, 51 mmols), was suspended in 50mL of 4M KOH (11.19g, 0.2mols) and heated to 70°C for 4 hours. Isobutyric Acid (1.38g, 15.8mmols), Isovaleric Acid (0.98g, 9.89mmols), 2-MethylButyric Acid (1.31g, 12.9mmols) and Valeric Acid (1.21g, 12.4mmols) were added in one portion and after 10 minutes of stirring Magnesium Chloride Hexahydrate (10.36g, 51mmols) in 5mL of water was slowly added to the resulting reddish orange suspension. After stirring for an additional hour to ensure homogeneity the burnt red suspension was added to 17g of Ground Corn Cob and dried in a vacuum oven prior to smell analysis.

### Smell Factor: 1

### Example 43

### MgCl₂ - mixed VFA - Pectin - KOH - Ground Corn

Pectin (10.00g, 51 mmols), was suspended in 50mL of 4M KOH (11.15g, 0.2mols) and heated to 70°C for 4 hours. Isobutyric Acid (1.38g, 15.8mmols), Isovaleric Acid (1.00g, 9.89mmols), 2-MethylButyric Acid (1.28g, 12.9mmols) and Valeric Acid (1.27g, 12.4mmols) were added in one portion and after 10 minutes of stirring Magnesium Chloride Hexahydrate (10.36g, 51mmols) in 5mL of water was slowly added to the resulting reddish orange suspension. After stirring for an additional hour to ensure homogeneity the burnt red suspension was added to 17g of Ground Corn and dried in a vacuum oven prior to smell analysis.

### Smell Factor: 1

### Example 44

### MgCl₂ - mixed VFA - Pectin - KOH - Cellulose

Pectin (9.91g, 51 mmols), was suspended in 50mL of 4M KOH (11.18g, 0.2mols) and heated to 70°C for 4 hours. Isobutyric Acid (1.40g, 15.8mmols), Isovaleric Acid (0.97g, 9.89mmols), 2-MethylButyric Acid (1.31g, 12.9mmols) and Valeric Acid (1.25g, 12.4mmols) were added in one portion and after 10 minutes of stirring Magnesium Chloride Hexahydrate (10.36g, 51mmols) in 5mL of water was slowly added to the resulting reddish orange suspension. After stirring for an additional hour to ensure homogeneity the burnt red suspension was added to 17g of Cellulose and dried in a vacuum oven prior to smell analysis.

### Smell Factor: 1

### Example 45

### MgCl₂ - mixed VFA - Pectin - KOH - Rice Flour

Pectin (9.99g, 51 mmols), was suspended in 50mL of 4M KOH (11.10g, 0.2mols) and heated to 70°C for 4 hours. Isobutyric Acid (1.39g, 15.8mmols), Isovaleric Acid (1.01g, 9.89mmols), 2-MethylButyric Acid (1.27g, 12.9mmols) and Valeric Acid (1.27g, 12.4mmols) were added in one portion and after 10 minutes of stirring Magnesium Chloride Hexahydrate (10.31g, 51mmols) in 5mL of water was slowly added to the resulting reddish orange suspension. After stirring for an additional hour to ensure homogeneity the burnt red suspension was added to 17g of Rice Flour and dried in a vacuum oven prior to smell analysis.

### Smell Factor: 1

### Example 46 (Comparative)

### Ammonia VFA (1:1, Neat)

Potassium Hydroxide (2.96g, 52mmols) was dissolved in 50mL of water. To this was added Isobutyric Acid (1.39g, 15.8mmols), Isovaleric Acid (1.01g, 9.89mmols), 2-MethylButyric Acid (1.31g, 12.9mmols) and Valeric Acid (1.27g, 12.4mmols) in one portion with an additional stirring for 10 minutes at room temperature. Ammonium Chloride Hexahydrate (2.78g, 52mmols) was dissolved in 5mL of water and added slowly to the reaction flask. The white suspension was stirred for 30 minutes, dried in a vacuum oven and analyzed for smell.

### Smell Factor: 8

### Example 47 (Comparative)

### K-VFA (1:1, Neat)

Potassium Hydroxide (2.95g, 52mmols) was dissolved in 50mL of water. To this was added Isobutyric Acid (1.39g, 15.8mmols), Isovaleric Acid (0.99g, 9.89mmols), 2-MethylButyric Acid (1.31g, 12.9mmols) and Valeric Acid (1.27g, 12.4mmols) in one portion with an additional stirring for 10 minutes at room temperature. The white suspension was dried in a vacuum oven and analyzed for smell.

### Smell Factor: 4

### Example 48 (Comparative)

### Na-VFA (1:1, Neat)

Sodium Hydroxide (2. 10g, 52mmols) was dissolved in 50mL of water. To this was added Isobutyric Acid (1.33g, 15.8mmols), Isovaleric Acid (1.00g, 9.89mmols), 2-MethylButyric Acid (1.29g, 12.9mmols) and Valeric Acid (1.27g, 12.4mmols) in one portion with an additional stirring for 10 minutes at room temperature. The white suspension was dried in a vacuum oven and analyzed for smell.

### Smell Factor: 4

### Example 49

### CaCl₂ - Isobutyric - CMC high viscosity - KOH

Sodium Carboxymethyl Cellulose - High Viscosity (2.03g, ~ 7.27mmols of COOH) was dissolved in 100mL of 2M KOH (11.21g, 0.2mols) over 4 hours. To this was added Isobutyric Acid (0.67mL, 7.27mmols) the resulting suspension was stirred for 20 minutes and to this was added Calcium Chloride Dihydrate (1.06g, 7.27mmols). The homogenous suspension was then stirred for an additional hour and dried in a vacuum oven prior to analyzing for smell.

### Smell Factor: 1

### Example 50

### MgCl₂ - Valeric/Isovaleric - CMC high viscosity - NaOH

Sodium Carboxymethyl Cellulose - High Viscosity (2.00g, ~ 7.27mmols of COOH) was dissolved in 100mL of 2M NaOH (7.95g, 0.2mols) over 4 hours. Valeric Acid (0.39mL, 3.6mmols) and Isovaleric acid (0.40mL, 3.6mmols) were added to the suspension which was stirred for 20 minutes and to this was added Magnesium Chloride Hexahydrate (1.44g, 7.27mmols). The homogenous suspension was then stirred for an additional hour and dried in a vacuum oven prior to analyzing for smell.

### Smell Factor: 1

### Example 51

### MgCl₂ - Isobutyric/Valeric - Alginic - KOH

Alginic Acid (10.01g, 51mmols) was dissolved in 100mL of 2M KOH (11.12g, 0.2mols) and heated to 70°C for 4 hours. To this was added Isobutyric Acid (2.37g, 25.5mmols) and Valeric Acid (2.78mL, 25.5mmols) the resulting orange/yellow suspension was stirred for 30 minutes and to this the Calcium Chloride Dihydrate (7.50g, 51mmols) was added. The homogenous suspension was stirred for an additional hour dried in the vacuum oven and analyzed for smell.

### Smell Factor: 1

### Example 52

### MgCl₂ - Isobutyric/Valeric/Isovaleric - Alginic - NaOH

Alginic Acid (9.93g, 51mmols) was dissolved in 100mL of 2M NaOH (8.05g, 0.2mols) and heated to 70°C for 4 hours. To this was added Isobutyric (1.58mL, 17mmols), Valeric Acid (1.86mL, 17mmols) and Isovaleric Acid (1.87mL, 17mmols) the resulting orange/yellow suspension was stirred for 30 minutes and to this the Magnesium Chloride Hexahydrate (10.33g, 51mmols) was added. The homogenous suspension was stirred for an additional hour dried in the vacuum oven and analyzed for smell.

### Smell Factor: 2

### Example 53

### CaCh - Valeric/Isovaleric/2-Methyl-Butyric - Pectin - KOH

Pectin (10.12g, 51 mmols), was suspended in 100mL of 2M KOH (11.15g, 0.2mols) and heated to 70°C for 4 hours. Valeric Acid (1.86mL, 17mmols), Isovaleric Acid (1.87mL, 17mmols) and 2-MethylButyric Acid (1.88mL, 17mmols) were added in one portion and after 10 minutes of stirring Calcium Chloride Dihydrate (7.49g, 51mmols) in 5mL of water was slowly added to the resulting reddish orange suspension. After stirring for an additional hour to ensure homogeneity the burnt red suspension was dried in a vacuum oven and analyzed for smell.

### Smell Factor: 1

### Example 54

### MgCl₂ - VFA Mix - Pectin - NaOH

Pectin (9.97g, 51 mmols), was suspended in 100mL of 2M NaOH (8.11g, 0.2mols) and heated to 70°C for 4 hours. Isobutyric Acid (1.46mL, 15.8mmols), Valeric Acid (1.36mL, 12.4mmols), Isovaleric Acid (1.09mL, 9.89mmols) and 2-MethylButyric Acid (1.41mL, 12.9mmols) were added in one portion and after 10 minutes of stirring Magnesium Chloride Hexahydrate (10.35g, 51mmols) in 5mL of water was slowly added to the resulting reddish orange suspension. After stirring for an additional hour to ensure homogeneity the burnt red suspension was dried in a vacuum oven and analyzed for smell.

### Smell Factor: 1

### Example 55

### Ca(OH)₂ - Isobutyric - Pectin - KOH

Pectin (10.00g, 51 mmols), was suspended in 100mL of 1M KOH (5.60g, 0.1mols) and heated to 70°C for 4 hours. Isobutyric Acid (4.73mL, 51mmols) was added in one portion and after 10 minutes of stirring Calcium Hydroxide (3.78g, 51mmols) was added to the resulting reddish orange suspension. After stirring for an additional hour to ensure homogeneity the burnt red suspension was dried in a vacuum oven and analyzed for smell.

### Smell Factor: 1

### Example 56

### Mg(OH)₂ - Isobutyric/2-Methyl-Butyric - Pectin - KOH

Pectin (10.11g, 51 mmols), was suspended in 100mL of 1M KOH (5.55g, 0.1mols) and heated to 70°C for 4 hours. Isobutyric Acid (2.37mL, 25.5mmols) and 2-Methyl Butyric Acid (2.82mL, 25.5mmols) were added in one portion and after 10 minutes of stirring Magnesium Hydroxide (2.98g, 51mmols) was added to the resulting reddish orange suspension. After stirring for an additional hour to ensure homogeneity the burnt red suspension was dried in a vacuum oven and analyzed for smell.

### Smell Factor: 1

### Example 57 (not according to the invention)

### CaCl₂ - VFA Mix - Polyacrylic acid - KOH

Polyacrylic Acid (50% solution, 7.54g, 51mmols), was dissolved in 100mL of 2M KOH (11.20g, 0.2mols). Isobutyric Acid (1.46mL, 15.8mmols), Valeric Acid (1.36mL, 12.4mmols), Isovaleric Acid (1.09mL, 9.89mmols) and 2-MethylButyric Acid (1.41mL, 12.9mmols) were added and subsequently followed by the addition of Calcium Chloride Dihydrate (7.49g, 51mmol) dissolved in 5mL of water. The resulting white suspension was stirred for an additional hour until homogenous, dried in a vacuum oven and analyzed for smell.

### Smell Factor: 1

### Example 58 (not according to the invention)

### MgCl₂ - Valeric - Polyacrylic acid - NaOH

Polyacrylic Acid (50% solution, 7.48g, 51mmols), was dissolved in 100mL of 2M NaOH (7.98g, 0.2mols). Valeric Acid (5.57mL, 51mmols) was added to the clear solution and followed by the drop wise addition of Magnesium Chloride Hexahydrate (10.29g, 51mmol) dissolved in 5mL of water. The resulting white suspension was stirred for an additional hour until homogenous, dried in a vacuum oven and analyzed for smell.

### Smell Factor: 1

### Example 59 (Comparative)

### MgCl₂ - VFA Mix -Tartaric Acid - KOH

Tartaric acid (7.66g, 51mmols) was dissolved in 100mL of water to which was slowly added KOH (11.2g, 0.2mols). After stirring for 20 minutes, Isobutyric Acid (1.46mL, 15.8mmols), Valeric Acid (1.36mL, 12.4mmols), Isovaleric Acid (1.09mL, 9.89mmols) and 2-MethylButyric Acid (1.41mL, 12.9mmols) were added to the clear solution. When the solution clarifies Magnesium Chloride Hexahydrate (10.37g, 51mmols) is added and subsequently a fine white precipitant forms. The white suspension was stirred for an additional hour until homogenous, dried in a vacuum oven and analyzed for smell.

### Smell Factor: 7

### Example 60 (Comparative)

### MgCl₂ - VFA Mix - Citric Acid - KOH

Citric acid monohydrate (10.7g, 51mmols) was dissolved in 100mL of water to which was slowly added KOH (11.2g, 0.2mols). After stirring for 20 minutes, Isobutyric Acid (1.46mL, 15.8mmols), Valeric Acid (1.36mL, 12.4mmols), Isovaleric Acid (1.09mL, 9.89mmols) and 2-MethylButyric Acid (1.41mL, 12.9mmols) were added to the clear solution. When the solution clarifies Magnesium Chloride Hexahydrate (10.35g, 51mmols) is added. The colorless solution was stirred for an additional hour until homogenous, dried in a vacuum oven and analyzed for smell.

### Smell Factor: 6

### Example 61 (Comparative)

### NH₄Cl - VFA Mix - KOH - Liquid

Potassium Hydroxide (2.99g, 52mmols) was dissolved in 100mL of water. To this was added Isobutyric Acid (1.46mL, 15.8mmols), Valeric Acid (1.36mL, 12.4mmols), Isovaleric Acid (1.09mL, 9.89mmols) and 2-MethylButyric Acid (1.41mL, 12.9mmols) in one portion with an additional stirring for 10 minutes at room temperature. Ammonium Chloride (2.77g, 52mmols) was dissolved in 5mL of water and added slowly to the reaction flask. The white suspension was stirred for 30 minutes and then analyzed for smell while still a liquid.

### Smell Factor: 4

### Example 62 (Comparative)

### VFA Mix - Liquid

Isobutyric Acid (1.46mL, 15.8mmols), Valeric Acid (1.36mL, 12.4mmols), Isovaleric Acid (1.09mL, 9.89mmols) and 2-MethylButyric Acid (1.41mL, 12.9mmols) were dissolved in 100mL of water and stirred for 10 minutes to ensure homogeneity. The colorless solution was then analyzed for smell while still a liquid.

### Smell Factor: 6

### Example 63

### MgCl₂ - VFA Mix - Pectin - KOH - Liquid

Pectin (10g, 51 mmols), was suspended in 100mL of 2M KOH (11.13g, 0.2mols) and heated to 70°C for 4 hours. Isobutyric Acid (1.46mL, 15.8mmols), Valeric Acid (1.36mL, 12.4mmols), Isovaleric Acid (1.09mL, 9.89mmols) and 2-MethylButyric Acid (1.41mL, 12.9mmols) were added in one portion and after 10 minutes of stirring Magnesium Chloride Hexahydrate (10.36g, 51mmols) in 5mL of water was slowly added to the resulting reddish orange suspension. After stirring for an additional hour to ensure homogeneity the burnt red suspension was then analyzed for smell while it was still a liquid.

### Smell Factor: 1

### Example 64

### ZnCl₂ - Isobutyric - CMC - KOH

Sodium Carboxymethyl Cellulose - High Viscosity (1.99g, ~ 7.27mmols of COOH) was dissolved in 100mL of 2M KOH (11.24g, 0.2mols) over 4 hours. To this was added Isobutyric Acid (0.67mL, 7.27mmols) the resulting suspension was stirred for 20 minutes and to this was added Zinc Chloride (0.99g, 7.27mmols). The homogenous suspension was then stirred for an additional hour and dried in a vacuum oven prior to analyzing for smell.

### Smell Factor: 1

### Example 65

### CuCl₂ - Isobutyric/Valeric - Alginic - NaOH

Alginic Acid (9.96g, 51mmols) was dissolved in 100mL of 2M NaOH (8.01g, 0.2mols) and heated to 70°C for 4 hours. To this was added Isobutyric Acid (2.37g, 25.5mmols) and Valeric Acid (2.78mL, 25.5mmols) the resulting orange/yellow suspension was stirred for 30 minutes and to this the Copper Chloride dihydrate (8.71g, 51mmols) was added. The homogenous suspension was stirred for an additional hour dried in the vacuum oven and analyzed for smell.

### Smell Factor: 1

### Example 66

### MnCl₂ - Valeric/Isovaleric/2-Methyl-Butyric - Pectin - KOH

Pectin (10.03g, 51 mmols), was suspended in 100mL of 2M KOH (11.26g, 0.2mols) and heated to 70°C for 4 hours. Valeric Acid (1.86mL, 17mmols), Isovaleric Acid (1.87mL, 17mmols) and 2-MethylButyric Acid (1.88mL, 17mmols) were added in one portion and after 10 minutes of stirring Manganese Chloride (6.41g, 51mmol) was slowly added to the resulting red/orange suspension. After stirring for an additional hour to ensure homogeneity the burnt red suspension was dried in a vacuum oven and analyzed for smell.

### Smell Factor: 1

### Example 67 (not according to the invention)

### FeCl₂ - VFA Mix - PAA - NaOH

Polyacrylic Acid (50% solution, 7.50g, 51mmols), was dissolved in 100mL of 2M NaOH (8.09g, 0.2mols). Isobutyric Acid (1.46mL, 15.8mmols), Valeric Acid (1.36mL, 12.4mmols), Isovaleric Acid (1.09mL, 9.89mmols) and 2-MethylButyric Acid (1.41mL, 12.9mmols) were added and subsequently followed by the addition of Ferrous Chloride tetrahydrate (10.16g, 51mmol). The resulting green/brown suspension was stirred for an additional hour until homogenous, dried in a vacuum oven and analyzed for smell.

### Smell Factor: 1

### Example 68

### ZnCl₂ - Isobutyric - CMC - KOH - Liquid

Sodium Carboxymethyl Cellulose - High Viscosity (1.97g, ~ 7.27mmols of COOH) was dissolved in 100mL of 2M KOH (11.2g, 0.2mols) over 4 hours. To this was added Isobutyric Acid (0.67mL, 7.27mmols) the resulting suspension was stirred for 20 minutes and to this was added Zinc Chloride (0.99g, 7.27mmols). The homogenous suspension was then stirred for an additional hour and analyzed for smell while still a liquid.

### Smell Factor: 1

### Example 69

### CuCl₂ - Isobutyric/Valeric - Alginic - NaOH - Liquid

Alginic Acid (10.06g, 51mmols) was dissolved in 100mL of 2M NaOH (8.03g, 0.2mols) and heated to 70°C for 4 hours. To this was added Isobutyric Acid (2.37g, 25.5mmols) and Valeric Acid (2.78mL, 25.5mmols) the resulting orange/yellow suspension was stirred for 30 minutes and to this the Copper Chloride dihydrate (8.72g, 51mmols) was added. The homogenous suspension was stirred for an additional hour and analyzed for smell while still a liquid.

### Smell Factor: 1

### Example 70

### MnCl₂ - Valeric/Isovaleric/2-Methyl-Butyric - Pectin - KOH - Liquid

Pectin (9.90g, 51 mmols), was suspended in 100mL of 2M KOH (11.22g, 0.2mols) and heated to 70°C for 4 hours. Valeric Acid (1.86mL, 17mmols), Isovaleric Acid (1.87mL, 17mmols) and 2-MethylButyric Acid (1.88mL, 17mmols) were added in one portion and after 10 minutes of stirring Manganese Chloride (6.42g, 51mmol) was slowly added to the resulting red/orange suspension. After stirring for an additional hour to ensure homogeneity the burnt red suspension was analyzed for smell while still a liquid.

### Smell Factor: 1

### Example 71 (not according to the invention)

### FeCl₂ - VFA Mix - PAA - NaOH - Liquid

Polyacrylic Acid (50% solution, 7.59g, 51mmols), was dissolved in 100mL of 2M NaOH (8.03g, 0.2mols). Isobutyric Acid (1.46mL, 15.8mmols), Valeric Acid (1.36mL, 12.4mmols), Isovaleric Acid (1.09mL, 9.89mmols) and 2-MethylButyric Acid (1.41mL, 12.9mmols) were added and subsequently followed by the addition of Ferrous Chloride tetrahydrate (10.14g, 51mmol). The resulting green/brown suspension was stirred for an additional hour until homogenous and then analyzed for smell while still a liquid.

### Smell Factor: 1

### BUTYRIC ACID EXAMPLES

### Example 72

### ZnCl₂ - Butyric - CMC - NaOH

Sodium Carboxymethyl Cellulose - High Viscosity (2.02g, ~ 7.27mmols of COOH) was dissolved in 100mL of 2M NaOH (8g, 0.2mols) over 4 hours. To this was added Butyric Acid (0.61mL, 7.27mmols) the resulting suspension was stirred for 20 minutes and to this was added Zinc Chloride (0.99g, 7.27mmols). The homogenous suspension was then stirred for an additional hour and dried in a vacuum oven prior to analyzing for smell.

### Smell Factor: 1

### Example 73

### CuCl₂ - Butyric - Alginic - KOH

Alginic Acid (9.97g, 51mmols) was dissolved in 100mL of 2M KOH (11.18g, 0.2mols) and heated to 70°C for 4 hours. To this was added Butyric Acid (4.68mL, 51mmols) the resulting orange/yellow suspension was stirred for 30 minutes and to this the Copper Chloride dihydrate (8.70g, 51mmols) was added. The homogenous suspension was stirred for an additional hour dried in the vacuum oven and analyzed for smell.

### Smell Factor: 2

### Example 74

### MgCl₂ - Butyric - Pectin - KOH

Pectin (10.05g, 51 mmols), was suspended in 100mL of 2M KOH (11.21g, 0.2mols) and heated to 70°C for 4 hours. Butyric Acid (4.68mL, 51mmols) was added in one portion and after 10 minutes of stirring Magnesium Chloride Hexahydrate (10.38g, 51mmol) was slowly added to the resulting red/orange suspension. After stirring for an additional hour to ensure homogeneity the burnt red suspension was dried in a vacuum oven and analyzed for smell.

### Smell Factor: 1

### Example 75

### CaCh - Butyric - Pectin - KOH

Pectin (10.1 1g, 51 mmols), was suspended in 100mL of 2M KOH (11.18g, 0.2mols) and heated to 70°C for 4 hours. Butyric Acid (4.68mL, 51mmols) was added in one portion and after 10 minutes of stirring Calcium Chloride dihydrate (7.51g, 51mmol) was slowly added to the resulting red/orange suspension. After stirring for an additional hour to ensure homogeneity the burnt red suspension was dried in a vacuum oven and analyzed for smell.

### Smell Factor: 1

### Example 76 (not according to the invention)

### MnCl₂ - Butyric - PAA - NaOH

Polyacrylic Acid (50% solution, 7.58g, 51mmols), was dissolved in 100mL of 2M NaOH (8.02g, 0.2mols). Butyric Acid (4.68mL, 51mmols) was added and subsequently followed by the addition of Manganese Chloride (6.41g, 51mmol). The resulting green/brown suspension was stirred for an additional hour until homogenous, dried in a vacuum oven and analyzed for smell.

### Smell Factor: 2

### Example 77

### ZnCl₂ - Butyric - CMC - NaOH - Liquid

Sodium Carboxymethyl Cellulose - High Viscosity (1.98g, ~ 7.27mmols of COOH) was dissolved in 100mL of 2M NaOH (7.95g, 0.2mols) over 4 hours. To this was added Butyric Acid (0.61mL, 7.27mmols) the resulting suspension was stirred for 20 minutes and to this was added Zinc Chloride (1.00g, 7.27mmols). The homogenous suspension was then stirred for an additional hour and then analyzed for smell while still a liquid.

### Smell Factor: 1

### Example 78

### MgCl₂ - Butyric - Pectin - KOH - Liquid

Pectin (10.04g, 51 mmols), was suspended in 100mL of 2M KOH (11.16g, 0.2mols) and heated to 70°C for 4 hours. Butyric Acid (4.68mL, 51mmols) was added in one portion and after 10 minutes of stirring Magnesium Chloride Hexahydrate (10.37g, 51mmol) was slowly added to the resulting red/orange suspension. After stirring for an additional hour to ensure homogeneity the burnt red suspension was analyzed for smell while still a liquid.

### Smell Factor: 2

### Example 79

### CaCh - Butyric - Pectin - KOH - Liquid

Pectin (10.01g, 51 mmols), was suspended in 100mL of 2M KOH (11.23g, 0.2mols) and heated to 70°C for 4 hours. Butyric Acid (4.68mL, 51mmols) was added in one portion and after 10 minutes of stirring Calcium Chloride dihydrate (7.54g, 51mmol) was slowly added to the resulting red/orange suspension. After stirring for an additional hour to ensure homogeneity the burnt red suspension was analyzed for smell while still a liquid.

### Smell Factor: 2

### Example 80 (not according to the invention)

### MnCl₂ - Butyric - PAA - NaOH - Liquid

Polyacrylic Acid (50% solution, 7.57g, 51mmols), was dissolved in 100mL of 2M NaOH (7.99g, 0.2mols). Butyric Acid (4.68mL, 51mmols) was added and subsequently followed by the addition of Manganese Chloride (6.41g, 51mmol). The resulting pink suspension was stirred for an additional hour until homogenous and then analyzed for smell while still a liquid.

### Smell Factor: 2

### Example 81 (Determination of Effect on Milk Production)

The purpose of this example was to determine the response of early lactation dairy cattle to the compound of Example 1 (with no carrier) as indicated by increased production of milk and milk components. 38 cows were selected, 19 in a controlled group, and 19 to be fed the product of Example 1. All animals were fed the same feed with the only difference being whether or not product of Example 1 was fed.

Composition of a robot fed pellet with rumen product of Example 1 was as follows:
Ground corn, 25.5%
Aminoplus (treated soybean meal), 23.25%
Corn gluten feed, 17.75%
Wheat mids, 11.7%
Rumen product of Example 1, 10%
Soybean meal, 4.6%
Molasses, 3.93%
Rumen inert fat, 2.75%
MetaSmart, 0.52%

Listed in Tables 1 and 2 are the typical ingredient profile of the bunk mix and the combined bunk mix and robot pellet. Ingredient composition of the diet of course will vary from cow to cow as the amount of pellets offered to each cow vary by the level of milk production and days in milk. Feedstuffs were analyzed for nutrient content prior to the start of the study.

**Table 1. Ingredient composition of bunk mix for lactating cows^{a}.**

| **Ingredient, % of dry matter** | **Lactating Diet** |
|---|---|
| Corn silage | 30.14 |
| Alfalfa silage | 34.45 |
| Wheat straw | 1.86 |
| Corn grain, ground high moisture | 10.99 |
| Partially mixed ration (PMR) Base | 6.77 |
| Soybean meal, solvent extracted, 48% CP | 5.96 |
| Ecker mix | 9.82 |

| | |
|---|---|
| ^{a} Diets fed *ad libitum.* | |

**Table 2 shows a typical composition of the diet (PMR plus robot fed pellet).**

| **Ingredient, % of dry matter** | **Control Diet** | **Treatment Diet** |
|---|---|---|
| Corn silage | 27.50 | 27.50 |
| Alfalfa silage | 20.39 | 20.39 |
| Alfalfa hay | 7.02 | 7.02 |
| Wheat straw | 1.60 | 1.60 |
| Corn grain, fine ground | 8.22 | 7.64 |
| Corn grain, high moisture, medium grind | 9.85 | 9.85 |
| Amino Plus^{®a} | 6.13 | 6.03 |
| Wheat mids | 5.39 | 4.79 |
| Soybean hulls | 4.70 | 4.70 |
| Corn gluten feed, dry | 3.19 | 3.18 |
| Soybean meal, solvent | 1.49 | 2.04 |
| Energy Booster^{®b} | 1.00 | 1.05 |
| Alfalfa meal, dehydrated | 0.89 | -- |
| Isoacid product of Example 1 | -- | 1.68 |
| Molasses | 0.65 | 0.58 |
| Sodium bicarbonate | 0.51 | 0.51 |
| Salt | 0.36 | 0.36 |
| Tallow, porcine | 0.29 | 0.29 |
| Trace minerals, vitamins, mycotoxin binder | 0.19 | 0.19 |
| MetaSmart^{®c} | 0.18 | 0.18 |
| AjiPro L^{®d} | 0.15 | 0.15 |
| Magnesium oxide | 0.14 | 0.14 |
| Calcium carbonate | 0.09 | 0.09 |
| Dicalcium phosphate | 0.01 | 0.01 |
| Amaferm^{®e} | 0.03 | 0.03 |

| | | |
|---|---|---|
| ^{a} Ag Processing, Inc., Omaha, NE USA ^{b} Milk Specialties Global, Eden Prairie, MN USA ^{c} Adessio, Commentry, France ^{d} Ajinomoto North America, Inc., Raleigh, NC 27610 ^{e} Biozyme Inc., St. Joseph, MO USA | | |

Treatments were delivered to cows and robot milkers by a pelleted grain mix. Eating times were once a day as determined by farm staff, with diets fed *ad libitum.* Feed refusals were removed once a day just prior to fresh feed delivery. Stalls were raked throughout the day as needed to remove feces and urine deposited on stall platforms.

Table 3 shows the effect of treatment on body weight and lactation performance of 19 cows assigned to the study.

**Table 3**

| **Item** | **Control** | **Rumen product** | **SEM** | **P-value** |
|---|---|---|---|---|
| Cows, n | 19 | 19 | | |
| Parity^{a} | 2.7 (1.1) | 3.1 (1.2) | | |
| Days in milk at start of study^{a} | 60.1 (24.4) | 60.2 (24.8) | | |
| Milk, lb/d | 112.5 | 114.7 | 2.6 | 0.38 |
| Energy-corrected milk^{b}, lb/d | 108.7 | 112.5 | 2.3 | 0.11 |
| Fat, lb/d | 3.71 | 3.91 | 0.08 | 0.01 |
| Protein, lb/d | 3.31 | 3.36 | 0.08 | 0.57 |
| Fat, % | 3.31 | 3.44 | 0.05 | 0.02 |
| Protein, % | 2.95 | 2.94 | 0.03 | 0.63 |
| Body weight, final, lb | 1584 | 1570 | 25 | 0.58 |

| | | | | |
|---|---|---|---|---|
| ^{a} 3.5% fat and 3.0% true protein | | | | |

Data was statistically analyzed using a model that included a covariate (average of the respective variable the week before cows started to receive their respective treatments) and treatment.

It should be noted that there was a slight bridging issue with pellets for the cows fed the pellet with the rumen product of Example 1 such that from time to time, some cows would not receive the recommended amount of pellets.

In this study, one control cow developed mastitis and was removed from the study. To keep the study balanced, the rumen product of Example 1 cow that was paired with the control cow that was removed from the study, was removed from the study as well.

Overall, cows fed the pellet with the rumen product of Example 1 produced more milk fat (*P*≤0.05) and tended to produced more energy corrected milk (*P*≤0.15). Increased milk fat production was the result of cows fed the rumen product diet producing milk with a higher fat content (*P*≤0.05).

Results of this study indicate that feeding the rumen product of Example 1 to dairy cattle increases fat production and tends to increase production of energy-corrected milk. It should be noted that production response to the rumen product of Example 1 may have been limited due to cows not receiving their full allotment of treatment pellets. Even so, the increase in milk production was statistically significant.

As can be seen, the volatile fatty acid effect on milk production in terms of enhancing it occurs with the non-smelly product of the present invention, indicating operability and proof of effectiveness for its intended use.

## Claims

1. A feed supplement for ruminants, swine and poultry comprising:
the reaction product of a water soluble polycarboxylic acid metal salt with pendant carboxylic acid groups and an isoacid selected from the group consisting of isobutyric, isovaleric, 2-methyl-butyric and valeric acids; wherein the water soluble polycarboxylic acid is selected from the group pectin, alginic acid, and carboxymethyl cellulose; and wherein the metal of the metal salt is selected from calcium, magnesium, zinc, manganese, copper and iron.

2. The feed supplement of claim 1 wherein the feed supplement is dried neat.

3. The feed supplement of claim 1 mixed with a common feed supplement carrier and dried.

4. The feed supplement of claim 1 wherein the feed supplement carrier is selected from the group consisting of com cobs, whey, fermentation by products, soybean flour and meal, and barley.

5. The feed supplement of claim 1 in liquid format.

6. A method of feeding ruminant animals, swine and poultry an energy source comprising:
reacting an isoacid selected from the group consisting of isobutyric, isovaleric, 2-methyl-butyric and valeric acids with a metal salt of a polycarboxylic acid with pendant carboxylic acid groups to provide a reduced odor reaction product; wherein the water soluble polycarboxylic acid is selected from the group pectin, alginic acid, and carboxymethyl cellulose; wherein the metal of the metal salt is selected from calcium, magnesium, zinc, manganese, copper and iron and
feeding the resulting reaction product to a ruminant, swine or poultry.

7. The method of claim 6 where the reaction product is fed to the swine or poultry in feed at a rate of 0.01 % to 1% by weight of the feed ration.

8. The method of claim 7 wherein the reaction product is fed to the swine or poultry in feed at a rate of 0.1% to 0.3% by weight of feed ration.

9. A process of producing a reduced odor feed supplement containing isoacids comprising: reacting an isoacid nutrient with a water soluble metal salt of a polycarboxylic acid having pendant carboxyl groups to provide a reduced odor isoacid feed supplement; wherein the water soluble polycarboxylic acid is selected from the group pectin, alginic acid, and carboxymethyl cellulose; wherein the isoacid nutrient is selected from the group consisting of isobutyric, isovaleric, 2-methyl-butyric and valeric acids; and wherein the metal of the metal salt is selected from calcium, magnesium, zinc, manganese, copper and iron.

## Patentansprüche

1. Futterzusatz für Wiederkäuer, Schweine und Geflügel, der Folgendes enthält:
das Reaktionsprodukt eines wasserlöslichen Polycarbonsäure-Metallsalzes mit seitenständigen Carbonsäuregruppen und einer Isosäure, die aus der Gruppe bestehend aus Isobuttersäure, Isovaleriansäure, 2-Methylbuttersäure und Valeriansäure ausgewählt ist; wobei die wasserlösliche Polycarbonsäure aus der Gruppe Pektin, Alginsäure und Carboxymethylcellulose ausgewählt ist; und wobei das Metall des Metallsalzes aus Calcium, Magnesium, Zink, Mangan, Kupfer und Eisen ausgewählt ist.

2. Futterzusatz nach Anspruch 1, wobei der Futterzusatz pur getrocknet ist.

3. Futterzusatz nach Anspruch 1 mit einem üblichen Futterzusatzträger gemischt und getrocknet.

4. Futterzusatz nach Anspruch 1, wobei der Futterzusatzträger aus der Gruppe bestehend aus Maiskolben, Molke, Fermentationsnebenprodukten, Sojamehl und -schrot und Gerste ausgewählt ist.

5. Futterzusatz nach Anspruch 1 in flüssiger Form.

6. Verfahren zum Füttern von Wiederkäuern, Schweinen und Geflügel mit einer Energiequelle, das Folgendes beinhaltet:
Umsetzen einer Isosäure, die aus der Gruppe bestehend aus Isobuttersäure, Isovaleriansäure, 2-Methylbuttersäure und Valeriansäure ausgewählt ist, mit einem Metallsalz einer Polycarbonsäure mit seitenständigen Carbonsäuregruppen, um ein geruchsreduziertes Reaktionsprodukt bereitzustellen; wobei die wasserlösliche Polycarbonsäure aus der Gruppe Pektin, Alginsäure und Carboxymethylcellulose ausgewählt ist; wobei das Metall des Metallsalzes aus Calcium, Magnesium, Zink, Mangan, Kupfer und Eisen ausgewählt ist, und
Verfüttern des resultierenden Reaktionsprodukts an einen Wiederkäuer, ein Schwein oder Geflügel.

7. Verfahren nach Anspruch 6, wobei das Reaktionsprodukt dem Schwein oder Geflügel im Futter in einer Menge von 0,01 bis 1 Gew.-% der Futterration zugeführt wird.

8. Verfahren nach Anspruch 7, wobei das Reaktionsprodukt dem Schwein oder Geflügel im Futter in einer Menge von 0,1 bis 0,3 Gew.-% der Futterration zugeführt wird.

9. Verfahren zur Herstellung eines Isosäuren enthaltenden geruchsreduzierten Futterzusatzes, das Folgendes beinhaltet:
Umsetzen eines Isosäuren-Nährstoffs mit einem wasserlöslichen Metallsalz einer Polycarbonsäure mit seitenständigen Carboxylgruppen, um einen geruchsreduzierten Isosäuren-Futterzusatz bereitzustellen; wobei die wasserlösliche Polycarbonsäure aus der Gruppe Pektin, Alginsäure und Carboxymethy-1-cellulose ausgewählt ist; wobei der Isosäurenährstoff aus der Gruppe bestehend aus Isobuttersäure, Isovaleriansäure, 2-Methylbuttersäure und Valeriansäure ausgewählt ist; und wobei das Metall des Metallsalzes aus Calcium, Magnesium, Zink, Mangan, Kupfer und Eisen ausgewählt ist.

## Revendications

1. Complément alimentaire pour ruminants, porcs et volailles comprenant :
le produit réactionnel d'un sel métallique d'acide polycarboxylique hydrosoluble comportant des groupes acide carboxylique pendants et d'un iso-acide sélectionné dans le groupe constitué des acides isobutyrique, isovalérique, 2-méthylbutyrique et
valérique ; l'acide polycarboxylique hydrosoluble étant sélectionné dans le groupe constitué de la pectine, de l'acide alginique et de la carboxyméthylcellulose ; et le métal du sel métallique étant sélectionné parmi le calcium, le magnésium, le zinc, le manganèse, le cuivre et le fer.

2. Complément alimentaire selon la revendication 1, le complément alimentaire étant séché à l'état pur.

3. Complément alimentaire selon la revendication 1 mélangé avec un support courant pour complément alimentaire et séché.

4. Complément alimentaire selon la revendication 1 dans lequel le support pour complément alimentaire est sélectionné dans le groupe constitué de la rafle de maïs, du lactosérum, de sous-produits de fermentation, de la farine et de la semoule de soja et de l'orge.

5. Complément alimentaire selon la revendication 1 sous forme liquide.

6. Méthode consistant à faire consommer à des ruminants, des porcs et des volailles une source d'énergie comprenant :
le fait de faire réagir un iso-acide sélectionné dans le groupe constitué des acides isobutyrique, isovalérique, 2-méthylbutyrique et valérique avec un sel métallique d'un acide polycarboxylique comportant des groupes acide carboxylique pendants pour produire un produit réactionnel à odeur réduite ; l'acide polycarboxylique hydrosoluble étant sélectionné dans le groupe constitué de la pectine, de l'acide alginique et de la carboxyméthylcellulose ; le métal du sel métallique étant sélectionné parmi le calcium, le magnésium, le zinc, le manganèse, le cuivre et le fer et
le fait de faire consommer à un ruminant, un porc ou une volaille le produit réactionnel ainsi obtenu.

7. Méthode selon la revendication 6 dans laquelle on fait consommer le produit réactionnel au porc ou à la volaille dans les aliments à un taux de 0,01 % à 1 % en poids de la ration alimentaire.

8. Méthode selon la revendication 7 dans laquelle on fait consommer le produit réactionnel au porc ou à la volaille dans les aliments à un taux de 0,1 % à 0,3 % en poids de la ration alimentaire.

9. Procédé de fabrication d'un complément alimentaire à odeur réduite contenant des iso-acides comprenant : le fait de faire réagir un nutriment iso-acide avec un sel métallique hydrosoluble d'un acide polycarboxylique comportant des groupes carboxyle pendants pour produire un complément alimentaire iso-acide à odeur réduite ; l'acide polycarboxylique hydrosoluble étant sélectionné dans le groupe constitué de la pectine, de l'acide alginique et de la carboxyméthylcellulose ; le nutriment iso-acide étant sélectionné dans le groupe constitué des acides isobutyrique, isovalérique, 2-méthylbutyrique et valérique ; et le métal du sel métallique étant sélectionné parmi le calcium, le magnésium, le zinc, le manganèse, le cuivre et le fer.
